# EUROPEAN PATENT APPLICATION

(11) **EP 4 534 045 A1**
(43) Date of publication of application: **09.04.2025**
(21) Application number: 23201286.4
(22) Date of filing: 02.10.2023
(51) Int. Cl.: A61F 2/07, A61F 2/89, A61F 2/915

(54) **GRAFT AND METHOD OF MAKING A GRAFT**

(71) Applicant: DSM IP Assets B.V., 6221 BE Maastricht (NL)
(72) Inventor: CHENG, Wei, 6221 BE Maastricht (NL); LINSSEN, Marc, 6221 BE Maastricht (NL)
(74) Representative: dsm-firmenich IP

(57) **Abstract**

A graft includes a reinforcing element defining a continuous tubular structure extending axially between first and second ends, and a first film layer fixed to the reinforcing element and extending around the tubular structure. The reinforcing element has a natural length between the first and second ends, and the reinforcing element has a plurality of gaps therethrough. The first film layer extends across at least some of the plurality of gaps, and the first film layer has a plurality of corrugations within the plurality of gaps when the graft is in an equilibrium state. The reinforcing element may be a stent in some configurations. A method of forming such a graft includes applying an axial force on the reinforcing element during manufacturing to change the length of the reinforcing element from the natural length thereof.

## Description

### FIELD OF THE INVENTION

The disclosure relates to grafts, methods of making grafts, and the use of such grafts to treat various medical conditions, such as aortic aneurysms or peripheral artery disease.

### BACKGROUND

Grafts are commonly used to treat diseased blood vessels and other tubular structures within the body. For example, a graft may be employed to reinforce or radially open blood vessels for the purpose of restoring or maintaining blood flow. Grafts may be implanted in the coronary, aortic and peripheral vasculature, neurovasculature, and in other bodily conduits such as the urinary tract, the bile duct, and the tracheo-bronchial tree.

Grafts typically comprise a metal stent covered by a fabric, film, and/or membrane. Various designs are possible for the metal stent, but they typically comprise a series of metal rings connected by metal bridges. The covering is typically a PTFE, an ePTFE (expanded PTFE) membrane, or a woven or knitted PET fabric.

Endovascular grafts require compressibility and flexibility to allow for proper positioning in the vasculature. Such grafts are either self-expandable or balloon expandable. A self-expandable graft expands upon removal of the constraint that keeps the graft from expanding. Balloon expandable grafts use a balloon to expand the graft. In either case, the graft must be compressible during delivery and positioning and expand to exert pressure on the vessel wall upon delivery. Since their inception, it has been desired to improve the flexibility and kink resistance of grafts. This has been somewhat achieved by improvements to the design of the metal stent or its material of construction. Nitinol is often a preferred material for a reinforcing element of such a graft due to its ability to withstand a significant amount of bending without permanent deformation.

The presence of the tubular sleeve, in contrast to a bare metal stent, is either for forming a closed conduit, such as in the case of treating a blood vessel aneurysm, like an abdominal aortic aneurysm (AAA) or thoracic aortic aneurysm (TAA), or for preventing unwanted tissue ingrowth into the lumen with time due to undesired conduit wall tissue remodeling stimulated by the metal stent. The presence of the graft material could even change stress distribution along the stent and minimize stent breakage in demanding applications like a long stent used in a superficial femoral artery. The mechanical behavior of stent grafts or covered stents, such as flexibility, bendability, conformability and kink-resistance, is important for their clinical performance, either in vascular vessels, or in other conduits in the body like biliary, gastrointestinal, tracheobronchial, and so on. The mechanical behavior of such covered stents is determined by the mechanical properties and structural characteristics of both the stent and the graft material, including their adjoining interaction details.

In order to have a flexible and kink-resistant stent graft that can conform to various curved shapes, the stent itself needs to be flexible enough to allow the intended bending. On the other hand, even if a stent is flexible, the combination with a graft material may not necessarily result in a flexible stent graft. Instead, the stent graft often becomes less flexible and easier to kink after combination with the graft material, for at least two principal reasons. First, the graft material may be less flexible, and the tubular graft material may be easy to kink. Second, the presence of the graft material may restrict the movement of the struts of the stent, and therefore reduces the flexibility of the assembly and makes the graft assembly easier to kink. These two factors work either independently or in combination, and the effect on flexibility may be stronger or weaker depending on the details of the stent design and the nature of the graft material.

The attachment of a graft material to a stent can be in the stent inner lumen, outer lumen, or both. For a fabric graft material, e.g., Dacron (PET) fabric, the attachment is mostly at the stent inner portion and is stitched to the stent using sutures. Despite the wide use of fabric graft material, the attachment to the stent using a suturing process is labor intensive and becomes less desired. In addition, due to the relatively large thickness of typical such fabric (> 60 µm), the fabric is not frequently used for small caliber stent grafts (e.g., with an outer diameter (OD) <12mm, particularly <8 mm.

Expanded polytetrafluoroethylene (ePTFE) a very common graft material, and the combination of ePTFE with a stent involves a sutureless process, e.g., by thermal bonding. An ePTFE graft material can be present in the inner and/or outer portion of a stent graft. The graft material may need to be present at least on the outer portion in order to securely attach the ePTFE material to the stent, creating a stent graft with covered inner and outer diameters (ID and OD). However, a stent graft with a graft material in the inner and outer portions typically restricts the strut movement, and thereby reduces flexibility and leads to easier kink formation. To alleviate this impairment to flexibility, a strip of helical ePTFE wrapping on the stent OD in combination with a flexible Z- or S-helical stent is applied to create full coverage of the inner diameter, with partial ePTFE coverage of the outer diameter. The advantage of this partial stent coverage is the exposure of the strut tip portion of the stent, to allow non-hindered movement during bending to maximally reserve the original flexibility of the stent.

Stent grafts with full OD coverage by ePTFE without ID coverage are also available. Such a configuration imposes less restriction to strut movement compared with a stent having full inner and outer coverage by a graft material. However, the weak bonding between the outer diameter of the graft material and the stent may present a risk of sliding against the stent with time. Additionally, ePTFE requires high thermal bonding temperatures and is susceptible to creep, which create disadvantages for such stent grafts.

There is a need for a stent graft with improved flexibility and kink-resistance, while achieving high radial strength. Radial strength is an important characteristic of stent grafts as it allows the stent graft to keep the tubular structure open during movement of the body and for the life of the stent graft. Radial strength is resistance to radial compression such as would be experienced by, for example, crimping. Kink-resistance is a resistance to a severe bending, which partially or completely obstructs flow through the stent graft. Kinking results in both compression of the stent graft on one side and extension on the other. Flexibility of a stent graft is exhibited by bending without kinking.

Certain prior art stent designs may have high radial strength but low flexibility. Even if the stent itself is designed to have reasonably good flexibility, the additional of a graft material results in a stent graft with less flexibility than the component stent itself. This is because the connections between metal rings of the stent push inward during bending and cause kinking. As a result, typical stents comprising several metal rings (crowns) and connections (bridges) are not ideal candidates for making stent grafts for applications where small caliber and high flexibility is required. On the other hand, stent grafts with high flexibility are possible, like GORE^{®} VIABAHN^{®} Endoprosthesis, which is essentially a helical wire superimposed with a secondary wavy pattern. However, such a stent graft achieves flexibility at the expense of radial strength.

A stent graft based on the typical stent structure but with connections (bridges) removed still exhibits kinking as the typically thin graft material on its own in a tubular form cannot bend significantly without kinking. One may consider a helical wire to reinforce such locations of the stent graft, but such reinforcement still possesses insufficient radial strength, and its incorporation complicates stent fabrication or assembly and increases costs. In addition, a helical wire requires sufficient length to achieve a sufficient number of helical turns and thereby meaningfully contribute to the mechanical properties of the stent graft. This length requirement reduces design flexibility and utility in small conduit applications. One may also consider woven or multi-layer materials, which may be more kink resistant than the industry-preferred ePTFE graft materials typically employed, but these materials are thicker, among other disadvantages, resulting in a larger device diameter that runs contrary to the ever-increasing demand for device miniaturization. These and other potential solutions may detrimentally increase the thickness of the stent graft, result in less desirable bio- and/or hemocompatibility, reduce flexibility, reduce radial strength, increase length and/or diameter of the stent graft, reduce design flexibility, or complicate assembly of the stent graft. The present disclosure is provided to address these and other disadvantages in existing grafts and methods for manufacturing the same. A full discussion of the features and advantages of the present invention is deferred to the following detailed description, which proceeds with reference to the accompanying drawings.

### BRIEF SUMMARY

General aspects of the present disclosure relate to grafts with one or more film layers that have corrugations, as well as methods of manufacturing a graft that include exerting an axial force on the stent and/or the graft to produce such corrugations.

Aspects of the disclosure relate to a graft that includes a reinforcing element defining a continuous tubular structure extending axially between first and second ends, and a first film layer fixed to the reinforcing element and extending around the tubular structure. The reinforcing element has a natural length between the first and second ends, and the reinforcing element has a plurality of gaps therethrough located between the first and second ends. The first film layer extends across at least some of the plurality of gaps, and the first film layer has a plurality of corrugations within the plurality of gaps when the graft is in an equilibrium state. The reinforcing element may be a stent in some configurations.

According to one aspect, the reinforcing element is compressed to a length shorter than the natural length of the reinforcing element when the graft is in the equilibrium state. In this configuration, the reinforcing element includes a helical wire forming a plurality of crowns, and the reinforcing element is configured such that a first crown of the plurality of crowns does not circumferentially overlap any adjacent portions of a second crown of the plurality of crowns when the reinforcing element is at the natural length.

According to another aspect, the reinforcing element is extended to a length longer than the natural length of the reinforcing element when the graft is in the equilibrium state. In this configuration, the reinforcing element may include a helical wire forming a plurality of crowns, and the reinforcing element is configured such that at least one of the crowns of the plurality of crowns circumferentially overlaps an adjacent portion of a second crown of the plurality of crowns when the reinforcing element is at the natural length.

According to a further aspect, the graft includes a second film layer fixed to the reinforcing element and extending around the tubular structure, and the second film layer extends across at least some of the plurality of gaps. In this configuration, the first film layer is an inner film layer disposed radially inward of the reinforcing element and the second layer is an outer film layer disposed radially outward of the reinforcing element. Additionally, in one configuration, both the first film layer and the second film layer have a plurality of corrugations within the plurality of gaps when the graft is in the equilibrium state.

According to yet another aspect, at least some of the plurality of gaps are open gaps that extend around at least a full circumference of the graft.

According to a still further aspect, at least some of the plurality of gaps are closed gaps that extend around less than a full circumference of the graft.

Additional aspects of the disclosure relate to method of manufacturing a corrugated graft, including positioning a reinforcing element on a mandrel to define a continuous tubular structure extending axially between first and second ends, and positioning a first film layer in contact with the reinforcing element on the mandrel and extending around the tubular structure. The reinforcing element has a natural length between the first and second ends, and the reinforcing element has a plurality of gaps therethrough located between the first and second ends. The first film layer is thermally bonded to the reinforcing element to fix the first film layer to the reinforcing element and form a graft, such that the first film layer extends across at least some of the plurality of gaps, and the length of the reinforcing element is reduced after the thermal bonding, optionally with the application of a compressive axial force. The reduction in length of the reinforcing element axially compresses the first film layer and forms a plurality of corrugations in the first film layer within the plurality of gaps when the graft is in an equilibrium state, creating a corrugated graft. The reinforcing element may be a stent in some configurations.

According to one aspect, a tensile axial force is exerted on the reinforcing element to increase the length of the reinforcing element to a modified length that is longer than the natural length prior to the thermal bonding. In this configuration, the plurality of corrugations are formed by the reinforcing element axially compressing the first film layer as the length of the reinforcing element decreases from the modified length toward its natural length. In one embodiment, the reinforcing element is in the modified length during the thermal bonding. In one embodiment, the length of the reinforcing element may be reduced by exerting a compressive axial force on the reinforcing element subsequent to the thermal bonding. In this configuration, when the corrugated graft is in the equilibrium state, an equilibrium length of the stent may remain longer than the natural length, or may be equal to or shorter than the natural length. Additionally, the reinforcing element may include a helical wire forming a plurality of crowns, wherein at least one of the crowns of the plurality of crowns circumferentially overlaps an adjacent portion of a second crown of the plurality of crowns when the reinforcing element is at the natural length.

According to another aspect, the length of the reinforcing element is reduced by exerting a compressive axial force on the reinforcing element after thermally bonding the first film layer to the reinforcing element. In this configuration, the plurality of corrugations are formed by axially compressing the first film layer when the compressive axial force reduces the length of the reinforcing element. Because the axial force is exerted on the reinforcing element after thermally bonding the first film layer to the reinforcing element, the axial force is exerted on both the reinforcing element and the first film layer. The thermal bonding may be conducted with the reinforcing element at its natural length. In this configuration, when the corrugated graft is in the equilibrium state, an equilibrium length of the reinforcing element may be shorter than the natural length. Additionally, the reinforcing element may include a helical wire forming a plurality of crowns, and a first crown of the plurality of crowns does not circumferentially overlap any adjacent portions of a second crown of the plurality of crowns when the reinforcing element is at the natural length.

According to a further aspect, the method includes positioning a second film layer in contact with the reinforcing element on the mandrel and extending around the tubular structure, and thermally bonding the second film layer to the reinforcing element to fix the second film layer to the reinforcing element, such that the second film layer extends across at least some of the plurality of gaps. In this configuration, the first film layer is an inner film layer disposed radially inward of the reinforcing element and the second layer is an outer film layer disposed radially outward of the reinforcing element. Reducing the length of the reinforcing element further axially compresses the second film layer and forms a plurality of corrugations in the second film layer within the plurality of gaps when the corrugated graft is in the equilibrium state. In one embodiment, thermally bonding the first film layer and the second film layer to the reinforcing element bonds the first film layer to the second film layer to fix the first and second film layers to the reinforcing element.

Other features and advantages of the disclosure will be apparent from the following description taken in conjunction with the attached drawings.

### BRIEF DESCRIPTION OF THE DRAWINGS

To allow for a more full understanding of the present disclosure, it will now be described by way of example, with reference to the accompanying drawings in which:
FIG. 1 is a plan view of one embodiment of a graft according to aspects of the disclosure;
FIG. 2 is a plan view of another embodiment of a graft according to aspects of the disclosure;
FIG. 3 is a plan view of another embodiment of a graft according to aspects of the disclosure;
FIG. 4 is a flowchart depicting one embodiment of a method of manufacturing a graft according to aspects of the disclosure;
FIG. 5 is a flowchart depicting another embodiment of a method of manufacturing a graft according to aspects of the disclosure;
FIG. 6 is a flowchart depicting another embodiment of a method of manufacturing a graft according to aspects of the disclosure;
FIG. 7 is a schematic cross-section view of a step in another embodiment of a method of manufacturing a graft according to aspects of the disclosure;
FIG. 8 is a schematic plan view of another step in the method of FIG. 7;
FIG. 9 is a schematic cross-section view of another step in the method of FIG. 7;
FIG. 10 is a schematic plan view of another step in the method of FIG. 7;
FIG. 11 is a schematic plan view of another step in the method of FIG. 7;
FIG. 12 is a schematic plan view of another step in the method of FIG. 7;
FIG. 13 is a schematic plan view of a stent used in a step in another embodiment of a method of manufacturing a graft according to aspects of the disclosure;
FIG. 14 is a schematic cross-section view of another step in the method of FIG. 13;
FIG. 15 is a schematic plan view of another step in the method of FIG. 13;
FIG. 16 is a schematic cross-section view of another step in the method of FIG. 13;
FIG. 17 is a schematic plan view of another step in the method of FIG. 13;
FIG. 18 is a schematic plan view of another step in the method of FIG. 13;
FIG. 19 is a perspective view of another embodiment of a graft according to aspects of the disclosure;
FIG. 20 is a perspective view of another embodiment of a graft according to aspects of the disclosure;
FIG. 21 is a perspective view of another embodiment of a graft according to aspects of the disclosure;
FIG. 22 is a perspective view of another embodiment of a graft according to aspects of the disclosure;
FIG. 23 is a perspective view of another embodiment of a graft according to aspects of the disclosure;
FIG. 24 is a perspective view of another embodiment of a graft according to aspects of the disclosure;
FIG. 25 is a perspective view of another embodiment of a graft according to aspects of the disclosure;
FIG. 26 is a perspective view of another embodiment of a graft according to aspects of the disclosure;
FIGS. 27A-27EE are plan views of portions of various embodiments of reinforcing elements, in the form of stents, that may be used in connection with a graft and/or a method according to aspects of the disclosure; and
FIG. 28 is a plan view of another embodiment of a graft according to aspects of the disclosure.

### DETAILED DESCRIPTION

While this invention is susceptible of embodiments in many different forms, there are shown in the drawings and will herein be described in detail example embodiments of the invention with the understanding that the present disclosure is to be considered as an exemplification of the principles of the invention and is not intended to limit the broad aspect of the invention to the embodiments illustrated. In the following description of various example structures according to the invention, reference is made to the accompanying drawings, which form a part hereof, and in which are shown by way of illustration various example devices, systems, and environments in which aspects of the invention may be practiced. It is to be understood that other specific arrangements of parts, example devices, systems, and environments may be utilized and structural and functional modifications may be made without departing from the scope of the present invention.

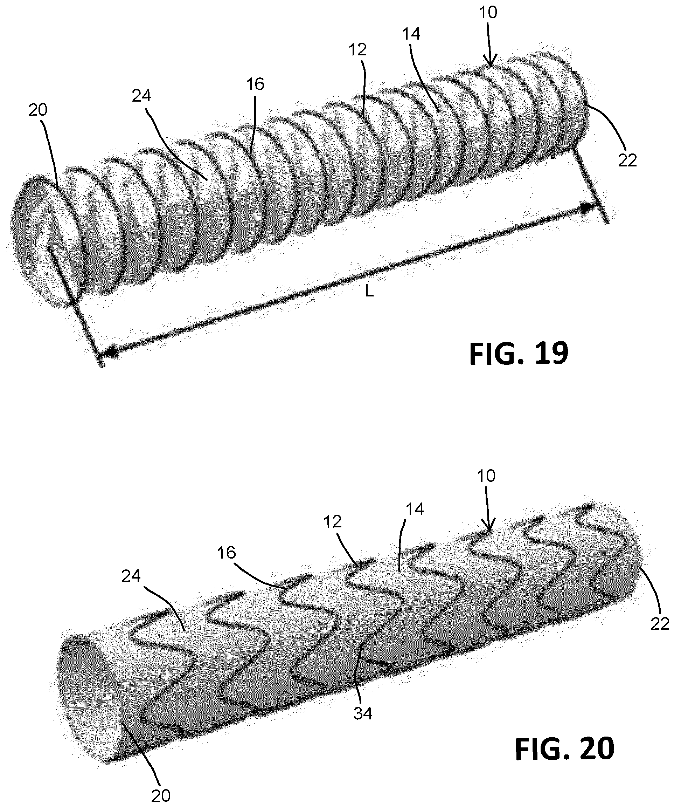

In one embodiment, a graft comprises a reinforcing element defining a continuous tubular structure extending axially between first and second ends, and a film including at least a first film layer fixed to the reinforcing element and extending around the tubular structure. The reinforcing element has a plurality of gaps therethrough, and the first film layer extends across at least some of the plurality of gaps. In this embodiment, the first film layer has a plurality of corrugations within the plurality of gaps when the graft is in an equilibrium state. In an embodiment, the reinforcing element is a stent. Stents may be formed of various different materials, including stainless steel, Nitinol or other shape memory alloy, fabric, silicone, etc. The reinforcing element has a natural length between the first and second ends, and in some embodiments, the length of the reinforcing element in the graft is different from the natural length. An example of the definition of the length L of the reinforcing element 12 is shown in FIG. 19. As used herein, the terms "natural length" and "equilibrium length" refer to the axial length of a component (the reinforcing element and the graft, respectively) when not acted on by an external force, although it is understood that some internal forces (e.g., residual stresses) may exist that affect the axial length of the component. Additionally, in one embodiment, the graft includes at least first and second film layers fixed to the reinforcing element, where the first film layer is an inner layer disposed radially inwardly of the reinforcing element, and the second film layer is an outer layer disposed radially outwardly of the reinforcing element.

The film may form both the inner diameter of the stent graft. The film may be formed from multiple pieces or layers, such as by bonding together multiple separate layers of film and/or by bonding together different sections of film at various interfaces. One exemplary method of forming the film is to helically wrap the material around a mandrel and bonding the overlapping sections together to form the film. The film is typically formed from a polymer material, such as PTFE, such as ePTFE, or UHMWPE (ultra-high molecular weight polyethylene). In an embodiment, the film is porous. In an embodiment, the film is microporous or nanoporous. In an embodiment the film is non-porous.

In one embodiment, the film comprises polyethylene, such as ultra-high molecular weight polyethylene (UHMWPE). For example, the film may be a microporous polyethylene film, such as a microporous UHMWPE film, also known as a UHMWPE membrane. A suitable commercially available UHMWPE film is Dyneema Purity^{®} Membrane from DSM. In an embodiment, the film has a thickness of from 5 to 60 µm, or from 5 to 30 µm. In an embodiment, multiple layers of microporous UHMWPE film are bonded together using heat (thermal lamination) to create the film. In such an embodiment, each layer may have a thickness of from 2.5 to 25 µm, such as about 15 µm. Both porous and non-porous materials may be used. It is understood that the film may or may not be made from a single material. In an embodiment, the film may have a high stiffness or tensile modulus, such as at least 100 MPa, before and/or after lamination is performed. It is understood that the bending stiffness and/or strength of the film may be controlled, in some embodiments, by using greater or fewer layers to form the film, as using more layers may increase bending stiffness and/or strength, and vice versa.

In an embodiment, the graft includes one or more reinforcing elements, such as a stent. The reinforcing element is fixed or bonded to the film such that the reinforcing element is substantially immobile relative to the film. The step of bonding is typically carried out by thermal lamination (i.e., heat bonding or thermal bonding). The step of bonding the reinforcing element may be performed at the same time as the thermal lamination step used to form the film. Thus, multiple layers of film may be supplied, and the one or more reinforcing elements positioned on the layers of film and then the thermal lamination step carried out in order to both form the film and bond the reinforcing element to the film. An outer film covering both the reinforcing element(s) and the inner film may also be bonded to the reinforcing element(s) and/or the inner film. In an embodiment, the reinforcing element(s) is/are positioned between two films and the thermal lamination bonds the three elements together.

In an embodiment, the reinforcing element is a stent. In an embodiment, the reinforcing element is a zigzag stent. In an embodiment, the reinforcing element is a helical wire. In an embodiment, the stent comprises a plurality of cylindrical elements, such as a plurality of bands or crowns of a zigzag stent. In an embodiment, the stent comprises a plurality of cylindrical elements connected by one or more bridges. Typical stent materials are generally suitable for the reinforcing element. Various embodiments of stents that are usable as reinforcing elements as described herein are illustrated in the drawings and described in greater detail herein.

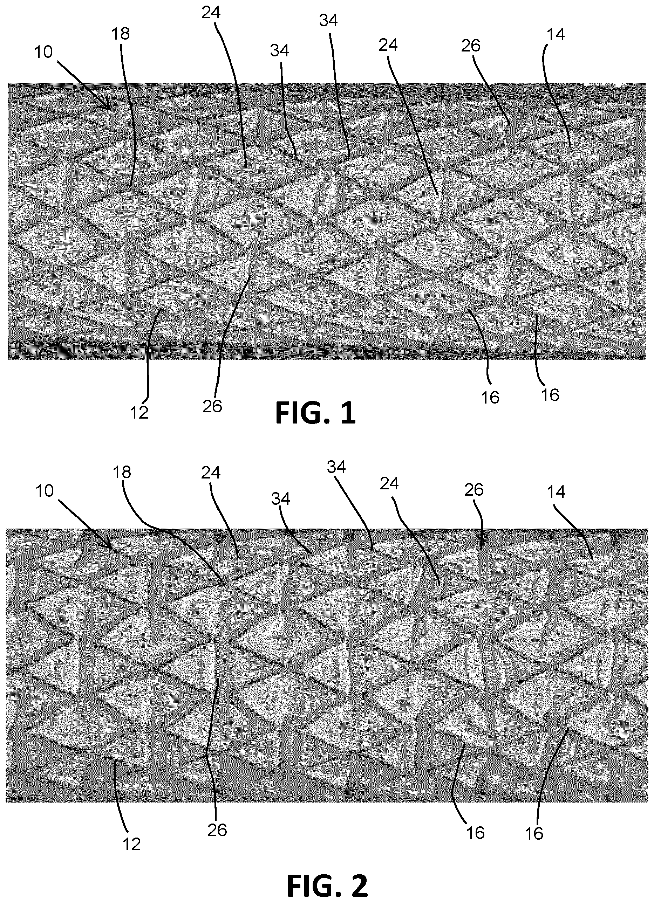

Referring to the drawings, and initially to FIGS. 1-3, three embodiments of grafts 10 are illustrated, each including a reinforcing element 12 in the form of a stent and a film 14 bonded to the reinforcing element 12, forming a tubular structure. The film 14 in these embodiments include at least inner and outer film layers disposed radially inwardly and radially outwardly of the reinforcing element 12, and which form inner and outer surfaces of the tubular structure. In an embodiment, the film 14 may include at least a third film layer formed inwardly or outwardly of the reinforcing element 12.

The embodiments of the grafts 10 in FIGS. 1 and 2 use similar reinforcing elements 12, which are helical stents having a plurality of crowns 16 extending helically around the tubular structure, with intermittent bridges 18 connecting adjacent crowns 16 together axially. The crowns 16 are formed having a plurality of alternating teeth 34, and the teeth 34 of adjacent crowns 16 extend in opposite directions relative to each other. The bridges 18 are formed between teeth 34 of adjacent crowns 18 that have points proximate to each other. Each reinforcing element 12 in FIGS. 1-2 has opposed first and second ends (not shown) and extends continuously and axially from the first end 20 to the second end 22. Additionally, each reinforcing element 12 defines a plurality of gaps 24 therethrough. The reinforcing elements 12 in FIGS. 1-2 form a mesh structure having closed gaps 24 that extend around less than a full circumference of the graft 10.

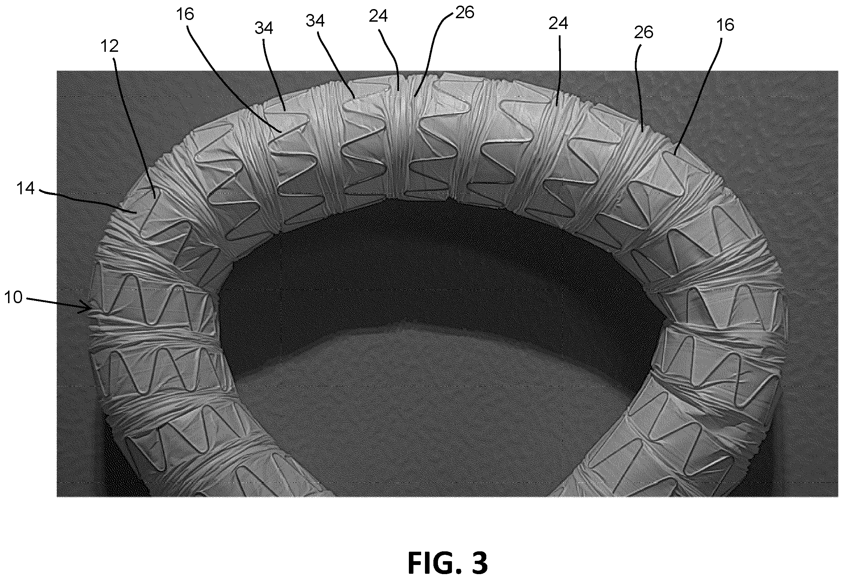

The embodiment of the graft 10 in FIG. 3 uses a reinforcing element 12 in the form of a helical stent having a plurality of crowns 16 extending helically around the tubular structure. The crowns 16 in this embodiment are not interconnected axially, and the crowns 16 are formed by a wire having a continuous helical shape. The reinforcing element 12 in FIG. 3 has opposed first and second ends (not shown) and extends continuously and axially from the first end to the second end. The gaps 24 defined by the reinforcing element 12 are open gaps. As used herein, an "open gap" is a gap that extends around at least a full circumference of the graft 10 within a continuous section of a reinforcing element 12, i.e., rather than a space between two adjacent reinforcing elements 12. It is understood that the aforementioned "full circumference of the graft" need not be defined by a plane perpendicular to the longitudinal axis of the graft 10. In one embodiment, the reinforcing element 12 has a single gap 24 that extends helically around the graft 10 over the entire or substantially entire length of the reinforcing element 12. Additionally, the spacing between the crowns 16 in the reinforcing element 12 of FIG. 3 is significantly larger than the spacing between the crowns 16 in FIGS. 1-2.

The film 14 forms a plurality of corrugations 26 within at least some of the gaps 24 in the embodiments of FIGS. 1-3. As used herein, the term "corrugations" refers to folds, bends, or other such deformations in the material of the film 14. The corrugations 26 in the films 14 in FIGS. 1-3 are formed within all of the gaps 24 between the ends 20, 22 of the reinforcing elements 12. The corrugations 26 in FIGS. 1-2 are formed as recesses each contained entirely within one of the gaps 24. The embodiment of FIG. 2 has corrugations 26 that are deeper than the corrugations 26 of the embodiment of FIG. 1, due to a greater degree of deformation during the corrugation forming, as described herein. The graft 10 in FIG. 3 has corrugations 26 that are annular or substantially annular. The corrugations 26 in the embodiments disclosed herein permit bending and flexing of the graft 10 to small bend radii without kinking of the film 14. FIG. 3 illustrates bending of the graft 10 while avoiding kinking. These corrugations 26 may also permit the length of the graft 10 to be stretched or extended during use or permit easier stretching or extending.

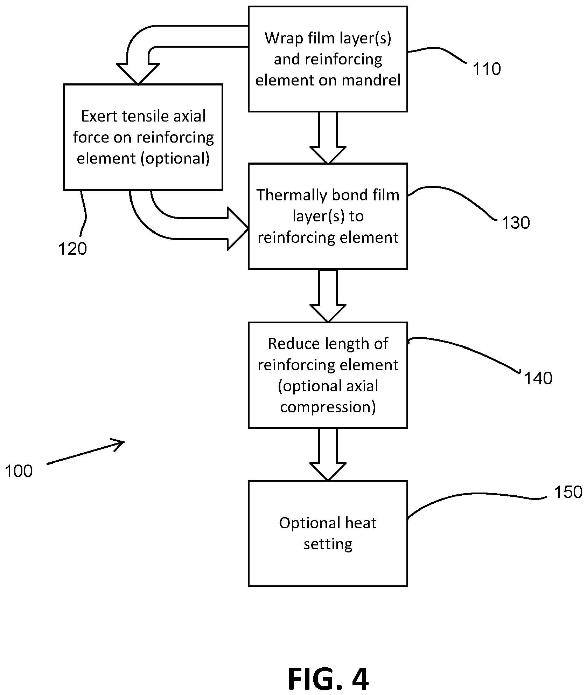

FIG. 4 illustrates one general embodiment of a method 100 of forming a graft 10 as disclosed herein. In this exemplary method, one or more film layers and one or more reinforcing elements 12 are wrapped or otherwise positioned on a mandrel for forming a graft 10, such that the mandrel extends through the inner lumen, at 110. An axial force is exerted on the reinforcing element 12 to change the length of the reinforcing element 12 at one or more points during the method 100, as discussed herein. This axial force may result in changing the reinforcing element 12 to a length different from the natural length, or returning the reinforcing element 12 to the natural length, or changing the reinforcing element 12 from a first length that is different from the natural length to a second length that is different from the natural length. In one embodiment, the axial force may be a tensile force that increases the length of the reinforcing element 12 to greater than the natural length, at optional step 120. In another embodiment, the axial force is a compressive force that decreases the length of the reinforcing element 12 to smaller than the natural length. The reinforcing element 12 and the film layers are thermally bonded together, e.g., by thermal lamination, to fix the film layers to the reinforcing element 12 and form the film 14 and the graft 10, at 130. In an embodiment, the thermal bonding may be performed by covering the layers of the film 14 and the reinforcing element 12 with a shrink tube of appropriate size to protect the assembly and placing the assembly into a preheated oven, such as at 127°C for 10 min. In another embodiment, a heat gun pre-treatment may be used to partially "lock" the assembly in place. Afterwards, the graft 10 is removed from the oven and cooled.

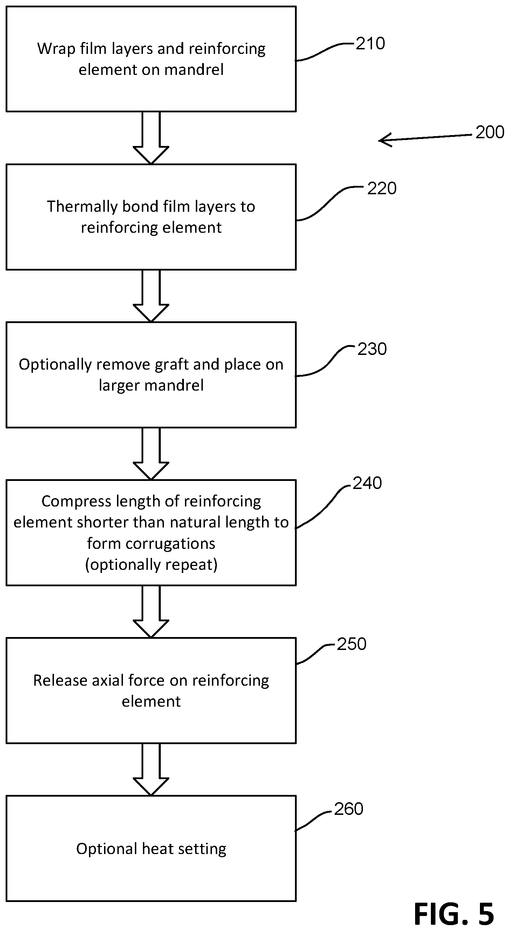

The thermal bonding 130 may be performed before or after the exertion of the axial force, in various embodiments, or the axial force may be applied both before and after the thermal bonding 130. If the thermal bonding 130 is performed first, then the axial force may be exerted on both the reinforcing element 12 and the film 14 together, which may axially deform both the reinforcing element 12 and the film 14. If the axial deformation is performed prior to the thermal bonding 130, then the axial force may be exerted only on the reinforcing element 12, and the axial deformation may be maintained throughout the thermal bonding 130. In a further embodiment, the axial deformation may be performed before, during, and after the thermal bonding 130. After the thermal bonding 130, the length of the reinforcing element 12 is reduced, at 140. This reduction in length 140 may be accomplished, in one embodiment, by exertion of a compressive axial force on the reinforcing element 12. In another embodiment, where a tensile axial force 120 is applied prior to and/or during thermal bonding 130, sufficient reduction in length may be achieved by spring force in the reinforcing element 12 when the axial force 120 is released. Corrugations 26 are formed in the film 14 during the length reduction 140 to form a corrugated graft 10, as described herein with respect to FIGS. 5-18. Finally, the graft 10 may optionally be subjected to one or more heat setting treatments. Thermal setting may be particularly useful in connection with reinforcing elements 12 having intrinsic compression resistance, e.g., those having closed gaps 24. Performing thermal setting in such a configuration permits the film 14 to be relaxed to allow the reinforcing element to relax back as close as possible to its natural length, thereby increasing flexibility in the final graft 10 created using this technique.

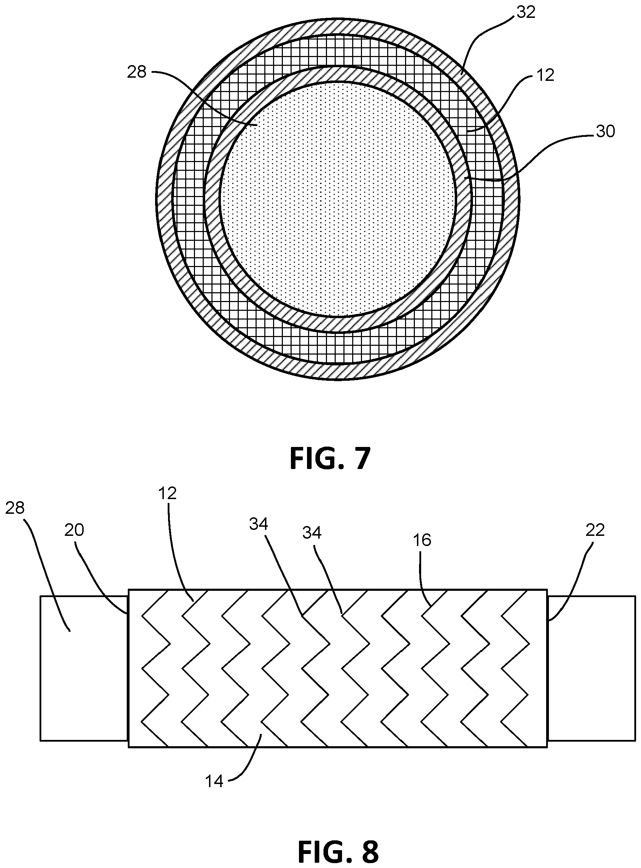

FIG. 5 illustrates another embodiment of a method 200 of forming a graft 10 as disclosed herein, where the axial force is exerted subsequent to thermal bonding. FIGS. 7-12 schematically illustrate some steps in this method 200. Inner and outer film layers 30, 32 and a reinforcing element 12 are wrapped or otherwise positioned on a mandrel 28 at 210, as shown in FIG. 7. This may be performed by first obtaining a film in the shape of a tube, which film will form the inner diameter of the graft 10. An example of this procedure is disclosed in U.S. Patent Application Publication No. 2017/0360584, which is incorporated by reference herein. To form the film into the shape of a tube, the film is cut to a selected size and wrapped around the outside diameter of the mandrel 28. The mandrel 28 may first be wrapped in a release layer, such as helically wrapped ePTFE tape, in order to facilitate removal of the film from the mandrel. A single layer or multiple layers of the film may be used to achieve the desired thickness. At least one layer of the film is positioned to form the inner film layer 30, and the reinforcing element 12 is placed on the mandrel 28 around the inner film layer 30. After the film layer 30 and the reinforcing element 12 are positioned on the mandrel 12, the film may be secured to the mandrel, such as securing it with wire, wrapping it in ePTFE tape, or using heat-shrink tubing. In addition, at least one layer of the film may then be wrapped around the reinforcing element 12 to form the outer film layer 32. The outer film 32 will form the outer diameter of the graft 10 after lamination. The outer film 32 may be formed similarly to the inner film 30, such as by wrapping the outer film material around the inner film 30 and reinforcing element 12 or placing layers of the material over the inner film 30 and reinforcing element 12 in a plurality of layers.

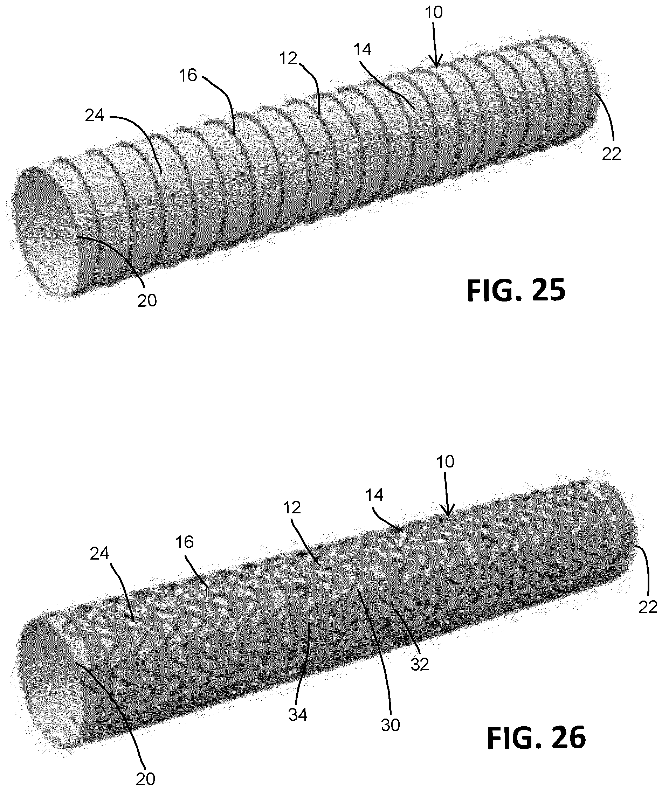

The mandrel 28 may have a constant or variable outer diameter to create tubes of various geometries such a tapered tube. The film layers 30, 32 may be treated, such as wet with isopropyl alcohol, to enable the film to better lay smoothly. In one embodiment, both the inner and outer film layers 30, 32 are continuous sleeves, but one or both such layers may have gaps therein in another embodiment, e.g., as in FIG. 26. In a further embodiment, either an inner film 30 or an outer film 32 may be omitted.

The film layers 30, 32 and the reinforcing element 12 are then thermally bonded together at 220 to form the multiple layers of film 14 together to form the graft 10 as a single tube. This step may be used to simultaneously bond the inner film 30 to the one or more reinforcing elements 12 and to the outer film 32. Bonding may be achieved by heat, such as in an air convection oven set at 150°C for 10 minutes. Once cool, the film 14 can be obtained by removing any inner and outer layers used in the process, such as an ePTFE tape release layer or any element used to secure the film in place on the mandrel 28. In the case of a microporous UHMWPE film, the time and temperature may be chosen such that the porous nature of the film 14 is not destroyed by thermal degradation. A suitable temperature and time combination may be chosen based on the specific character of the material. It is understood that the thermal bonding may be performed in a single step or multiple steps, e.g., by bonding the inner film 30 to the reinforcing element 12 in a first step and then applying and bonding the outer film 30 to the inner film 30 and the reinforcing element 12 in a second step. FIG. 8 illustrates the graft 10 after assembly and thermal bonding.

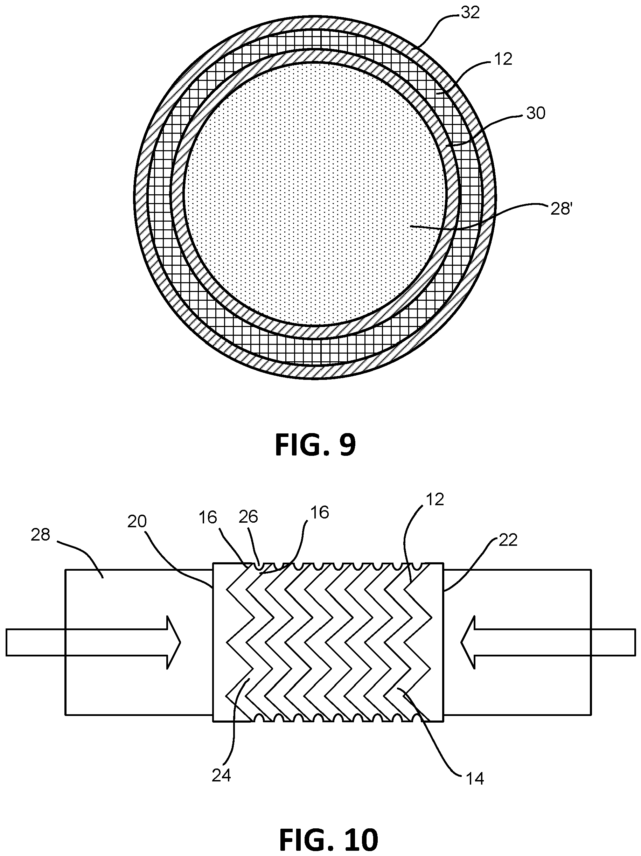

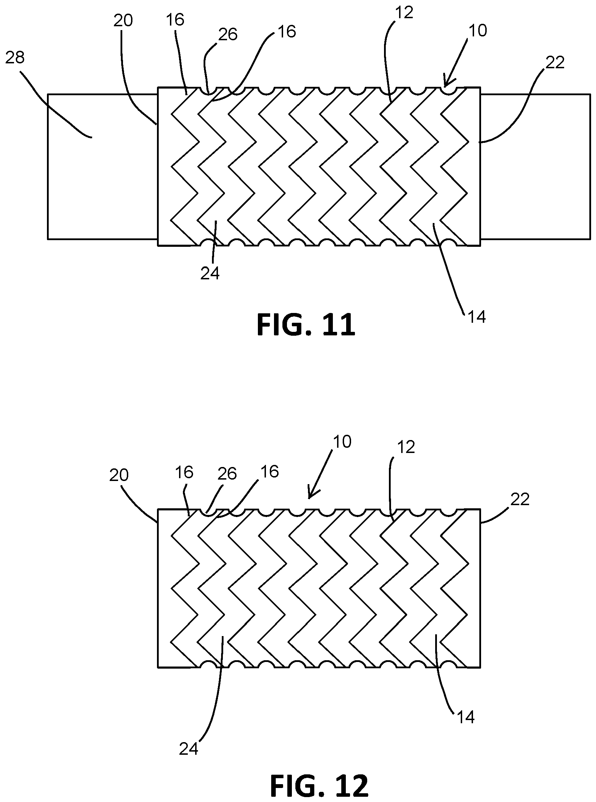

After thermal bonding 220, the graft 10 may optionally be removed from the original mandrel 28 and placed on a larger diameter mandrel 28' at 230. FIG. 9 illustrates the graft 10 positioned on a larger mandrel 28'. The graft 10 is then subjected to an axial compressive force at 240, which shortens the length of the reinforcing element 12 relative to the natural length. The graft 10 may be subjected to force either by hand or by machine (e.g., machine-controlled clamps), and the graft 10 is compressed to the point that strong resistance is experienced. This compression generates corrugations 26 in the gaps 24 between the crowns 16 of the reinforcing element 12, as illustrated in FIG. 10. Optionally, the compression may be released and repeated one or more times. The compression may be performed while the graft 10 is on the original mandrel 28, the larger mandrel 28', or in a different position (e.g., after removal of the mandrel). The axial force on the graft 10 is then released at 250, and the graft 10 expands its length somewhat, as shown in FIG. 11, to reach an equilibrium length. The equilibrium length of the graft 10 may be such that the length of the reinforcing element 12 remains shorter than the natural length, as shown in FIG. 11, or the reinforcing element 12 may return to its natural length in another embodiment. The graft 10 may then optionally be subjected to a heat setting treatment, at 260, which may be performed on the mandrel 28, 28' or after removal. The completed graft 10 is then removed from the mandrel 28, 28' and is complete, as shown in FIG. 12.

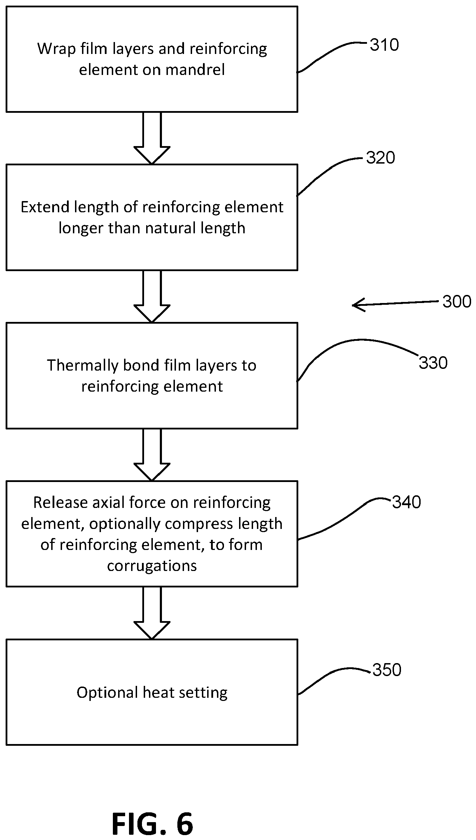

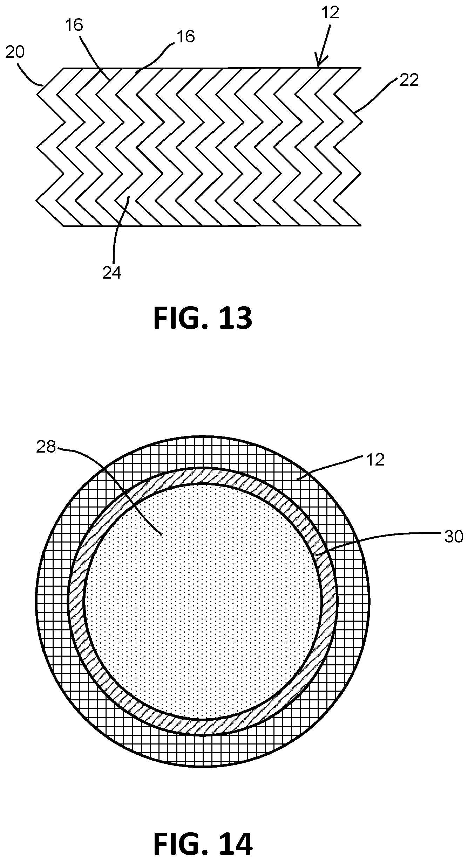

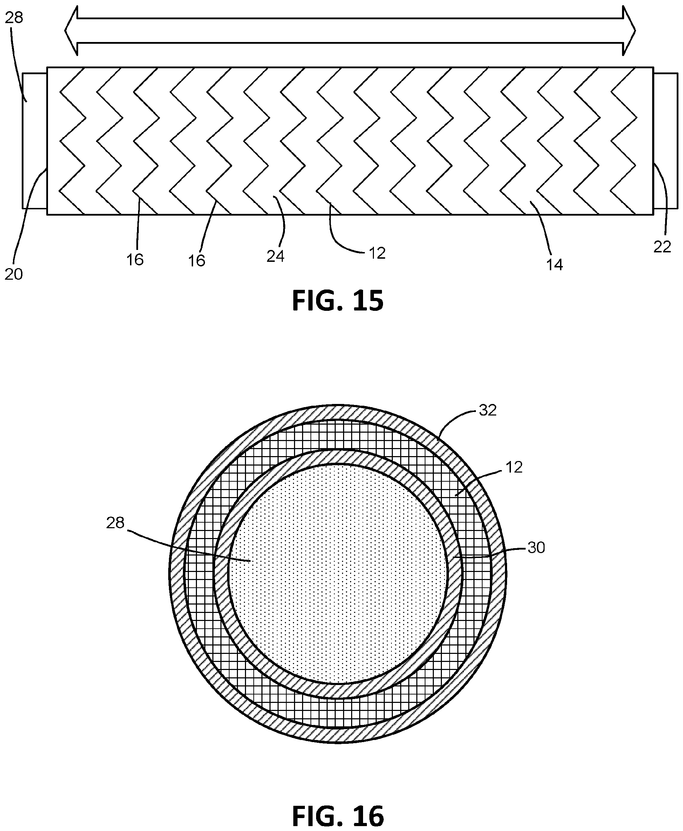

FIG. 6 illustrates another embodiment of a method 300 of forming a graft 10 as disclosed herein, where the axial force is exerted prior to and during thermal bonding. FIGS. 13-18 schematically illustrate some steps in this method 300. FIG. 13 illustrates the reinforcing element 12 prior to assembly. Inner and outer film layers 30, 32 and a reinforcing element 12 are wrapped or otherwise positioned on a mandrel 28 at 310, which may be performed as described above with respect to FIG. 5. In this embodiment, the inner film layer 30 and the reinforcing element 12 are first positioned on the mandrel 28, as shown in FIG. 14. The reinforcing element 12 is then subjected to an axial tensile force at 320, which increases the length of the reinforcing element 12 relative to the natural length, as shown in FIG. 15. The axial force may be exerted using a technique such as described above with respect to FIG. 5. In the embodiment illustrated in FIGS. 13-18, the reinforcing element 12 is axially stretched prior to wrapping the outer film layer 32. The inner layer 30 and the extended reinforcing element 12 are fixed onto the mandrel 28 either free of extra fixation assistance (e.g., relying solely on the friction force between the reinforcing element 12 and the inner layer 30) or with extra temporary fixations (e.g., small clamps present in the ends 30, 32 of the reinforcing element 12 or any desired locations along the length thereof). The former fixation mechanism works well with reinforcing elements 12 with small restoring force after stretching, and the latter may be needed with reinforcing elements 12 with higher restoring force after stretching.

FIG. 16 illustrates the wrapping of the outer film layer 32. The film layers 30, 32 and the reinforcing element 12 are then thermally bonded together at 330 to form the multiple layers of film 14 together to form the graft 10 as a single tube. The thermal bonding may also be performed as described herein with respect to FIG. 5, and it is understood that the axial force may be maintained on the reinforcing element 12 during the thermal bonding 330.

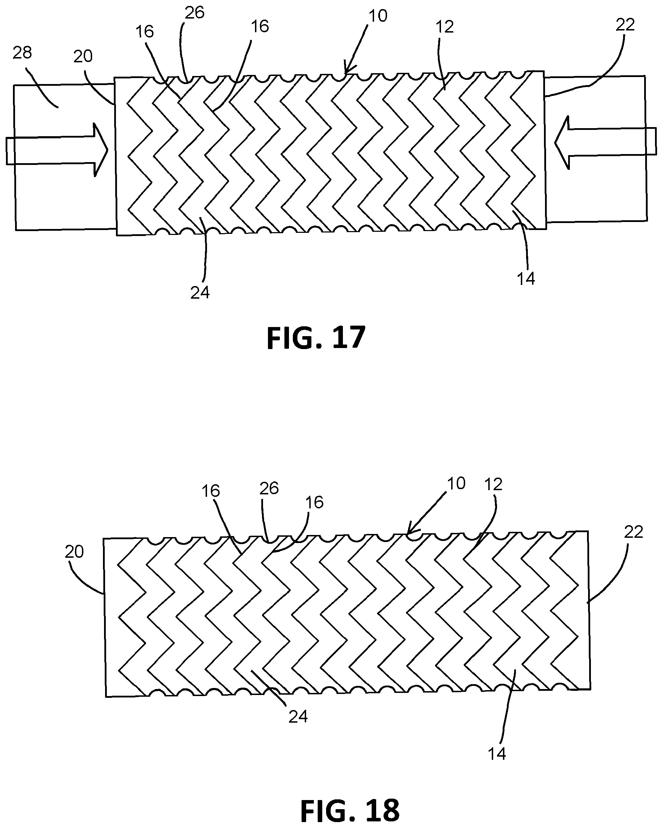

After thermal bonding 330, the tensile axial force on the reinforcing element 12 is released at 340, and the graft 10 may reduce its length somewhat, as shown in FIG. 17, to reach an equilibrium length. An axial compressive force may be exerted on the reinforcing element 12, at 340, to (further) reduce the length of the reinforcing element 12. This reduction in length of the graft 10 forms corrugations 26 in the film 14 in the gaps 24 between the crowns 16 of the reinforcing element 12, as illustrated in FIG. 17, which depicts exertion of a compressive axial force on the reinforcing element 12. In an embodiment with a relatively stiff film 14, the axial compression may be necessary to form the corrugations 26. Alternately, if the film 14 has lower stiffness and the reinforcing element 12 has sufficient spring force, the retraction force of the reinforcing element 12 may be sufficient to create corrugations 26. The equilibrium length of the graft 10 may be such that the length of the reinforcing element 12 remains longer than the natural length, as shown in FIG. 18, or the reinforcing element 12 may return to its natural length in another embodiment. In an embodiment where an axial compressive force is exerted on the reinforcing element 12, the final length of the reinforcing element 12 may be less than the natural length of the reinforcing element 12. The graft 10 may then optionally be subjected to a heat setting treatment, at 350, which may be performed on the mandrel 28 or after removal. The completed graft 10 is then removed from the mandrel 28 and is complete, as shown in FIG. 18.

In another embodiment, the method 200 of FIGS. 5 and 7-12 may also include axially stretching the reinforcing element 12 as described with respect to the method 300 of FIGS. 6 and 13-18. For example, the reinforcing element 12 may be axially stretched prior to and during the thermal bonding 220, as shown in FIG. 15, and then subsequently compressed. This technique can be used to create additional and/or deeper corrugations 26 and/or to create a graft 10 where the reinforcing element 12 has a length greater than or equal to the natural length, while also using axial compression for forming the corrugations 26. Similarly, an axial compression step (which may include placing the graft 10 on a larger mandrel 28' as shown in FIGS. 9-10) may be used in the method 300 of FIGS. 6 and 13-18. This may be performed after step 340 and may be used to assist in creating the corrugations 26 and/or to create a graft 10 where the reinforcing element 12 has a length less than or equal to the natural length. This additional step may also be useful when a very stiff film 14 is used, which may be difficult to form corrugations 26 due solely to the compressive force exerted by the reinforcing element 12 returning toward the natural length.

Selection of the method 200 of FIGS. 5 and 7-12 or the method 300 of FIGS. 6 and 13-18 may be performed based on the configuration of the reinforcing element 12. For example, the method 200 of FIGS. 5 and 7-12 may be advantageous when performed using a reinforcing element 12 with sparsely spaced crowns 16, which provides room for compression of the length of the reinforcing element 12 between the crowns 16. In one example, the method 200 of FIGS. 5 and 7-12 may be used with a reinforcing element 12 where one or more of the crowns 16 (or substantially all of the crowns 16) do not circumferentially overlap any adjacent portions of adjacent crowns 16 of the plurality of crowns 16 when the reinforcing element 12 is at the natural length. In this context, a "circumferential overlap" refers to both structures intersecting a plane extending radially, i.e., perpendicular to the longitudinal axis of the reinforcing element 12. FIG. 8 illustrates a reinforcing element 12 that meets these criteria. Conversely, the method 300 of FIGS. 6 and 13-18 may be advantageous when performed using a reinforcing element 12 with closely spaced crowns 16, where extension is more feasible than compression. In one example, the method 300 of FIGS. 6 and 13-18 may be used with a reinforcing element 12 where one or more of the crowns 16 (or substantially all of the crowns 16) circumferentially overlaps an adjacent portion of an adjacent crown 16 when the reinforcing element 12 is at the natural length. In another example, the method 300 of FIGS. 6 and 13-18 may be used with a reinforcing element 12 that uses closed gaps 24. FIG. 13 illustrates a reinforcing element 12 that meets these criteria.

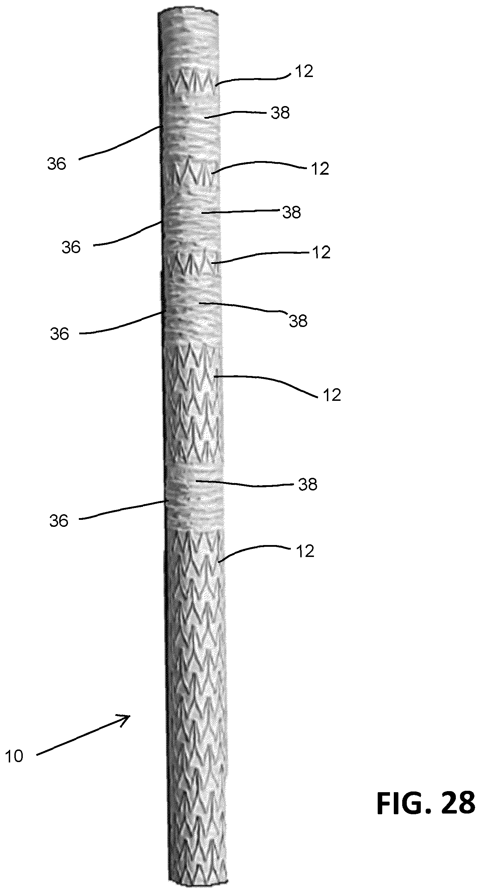

In a further embodiment, a graft 10 may be formed using multiple reinforcing elements 12 positioned along the length of the graft 10, with some or all of the reinforcing elements 12 having corrugations 26 within the gaps 24 therein. The graft 10 may also include corrugations in sections of the film between the reinforcing elements 12. FIG. 28 illustrates one such embodiment, where the graft 10 includes multiple reinforcing elements 12 with film sections 36 between the reinforcing elements, and additional corrugations 38 in the film sections 36. It is understood that the graft 10 in FIG. 28 may include corrugations 26 within the reinforcing elements 12 as described herein.

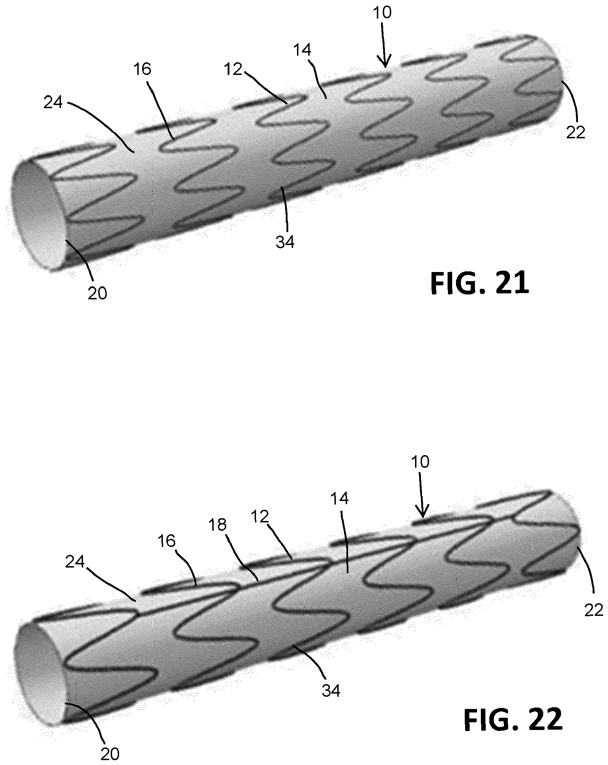

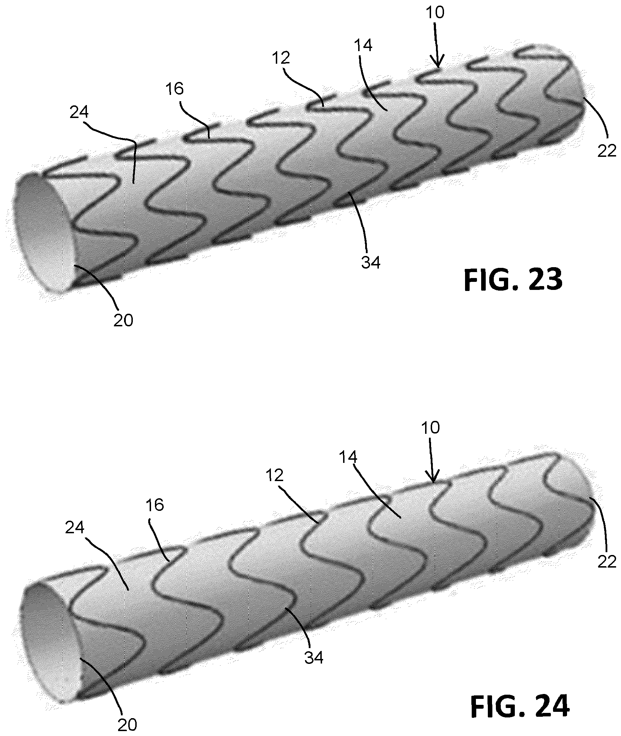

FIGS. 19-26 illustrate additional embodiments of grafts 10 that may be subjected to the methods 100, 200, 300 described herein to produce a graft 10 with corrugations 26 as disclosed herein. The grafts 10 in FIGS. 19-26 all utilize helical stents as reinforcing elements 12 and include films with both inner and outer layers. These reinforcing elements 12 have different configurations. For example, the reinforcing element 12 of FIG. 22 includes bridges 18 between the crowns 16. As another example, the reinforcing elements 12 of FIGS. 19 and 25 have straight helical crowns 16 having no teeth 34. As a further example, the reinforcing elements 12 of FIGS. 20-24 and 26 have teeth 34 where the teeth 34 of adjacent crowns 16 extend in the same directions relative to each other. As yet another example, the graft 10 of FIG. 26 uses an outer film layer 32 in the form of a helical wrap, rather than a tubular structure.

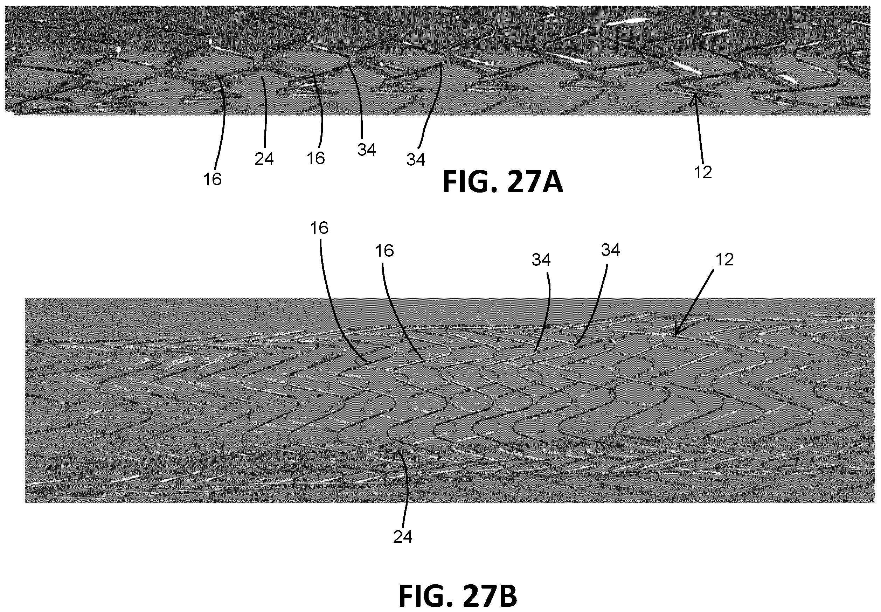

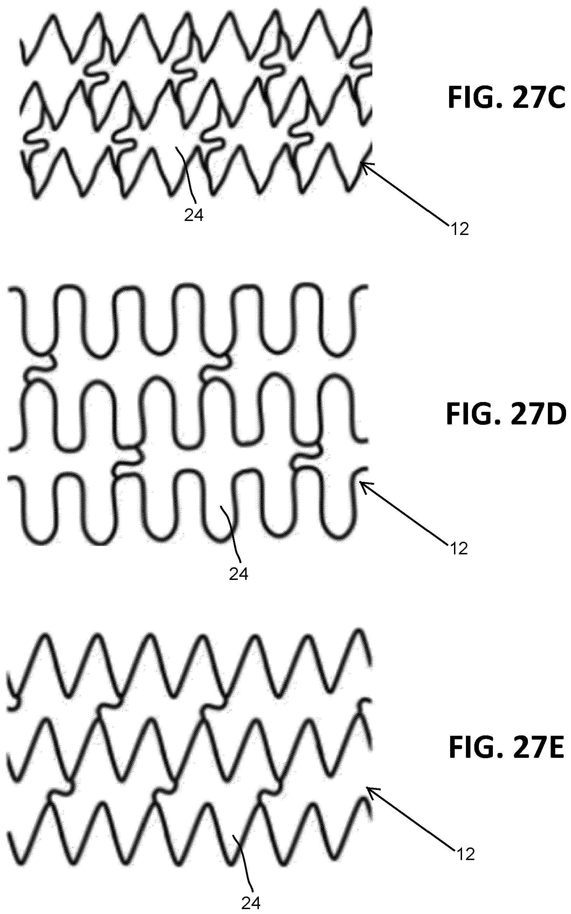

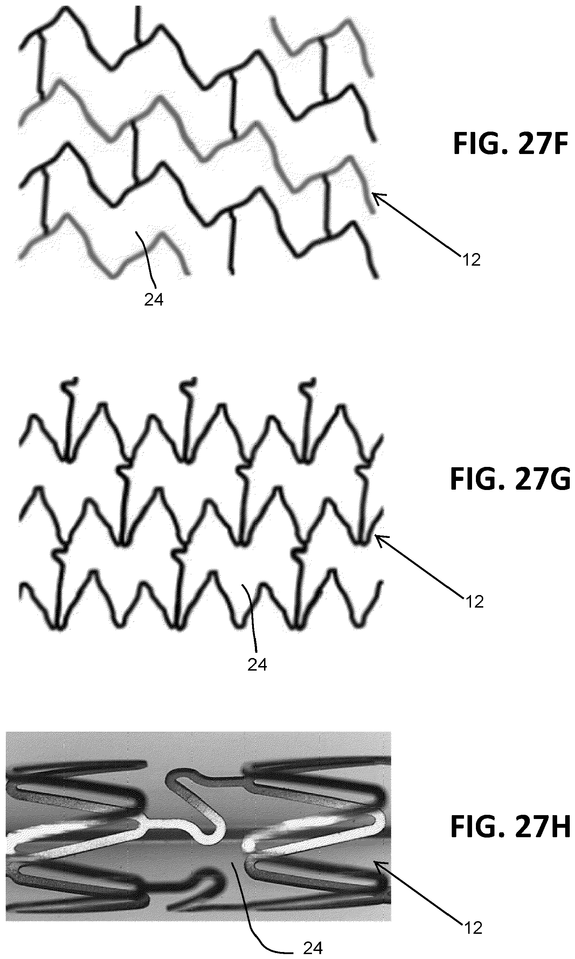

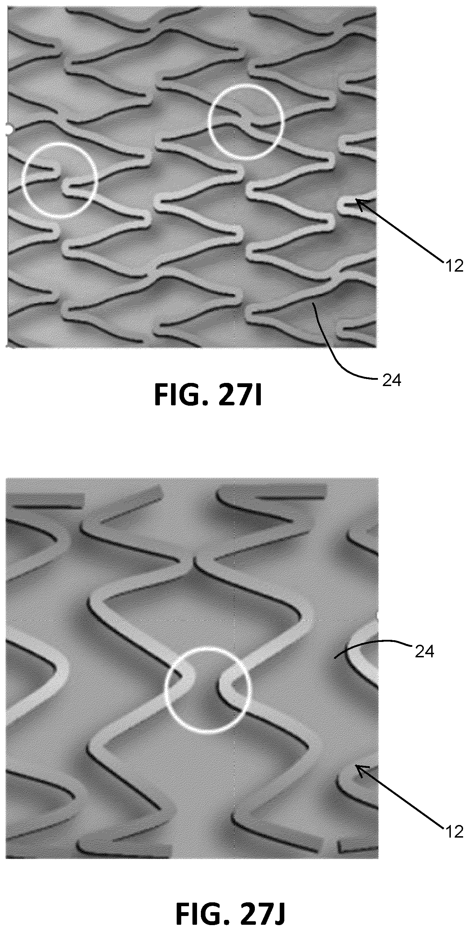

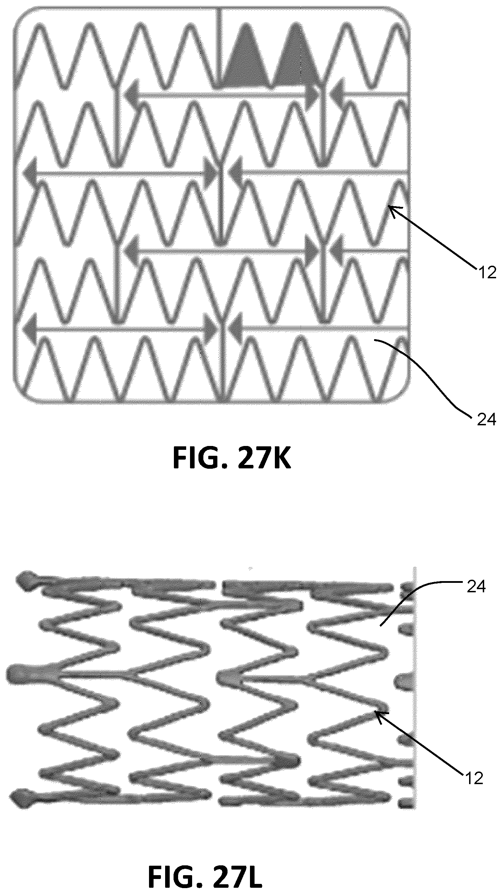

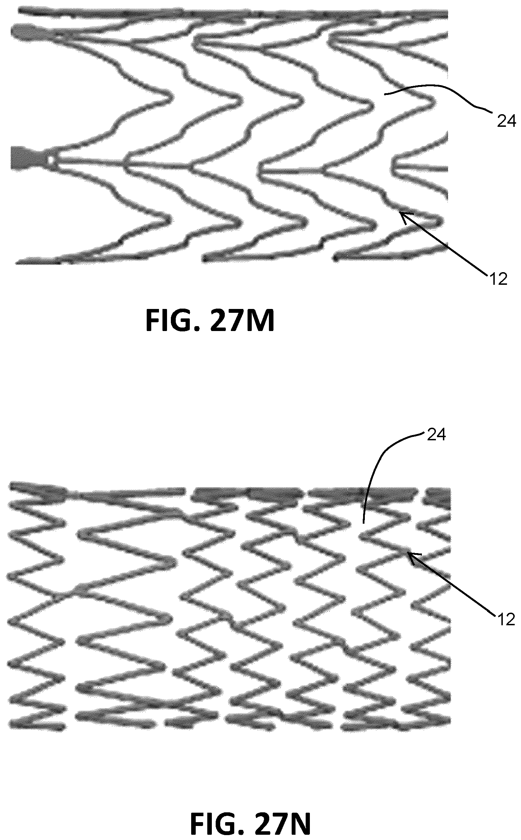

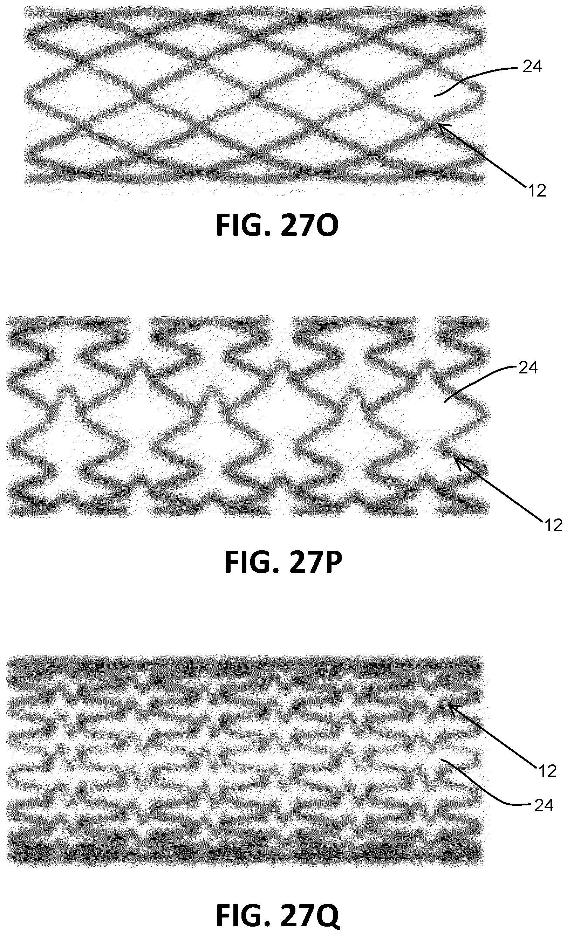

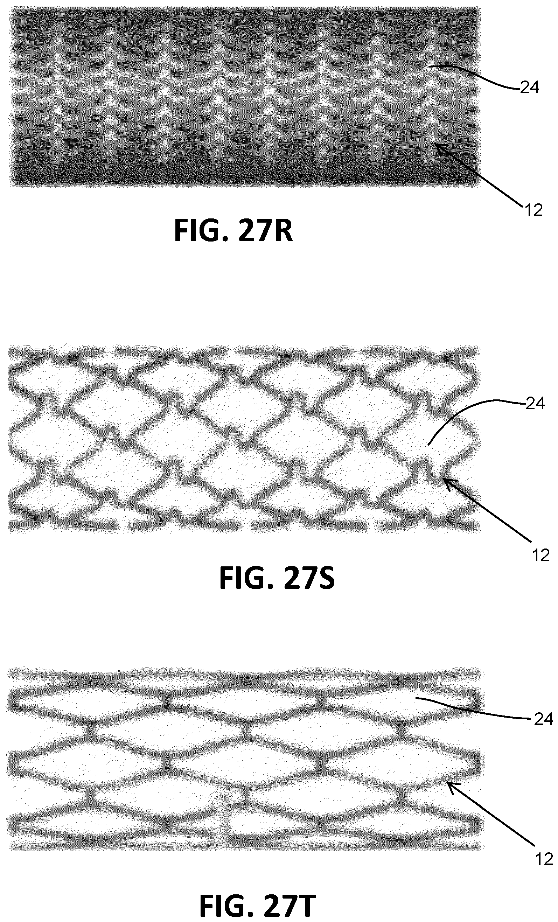

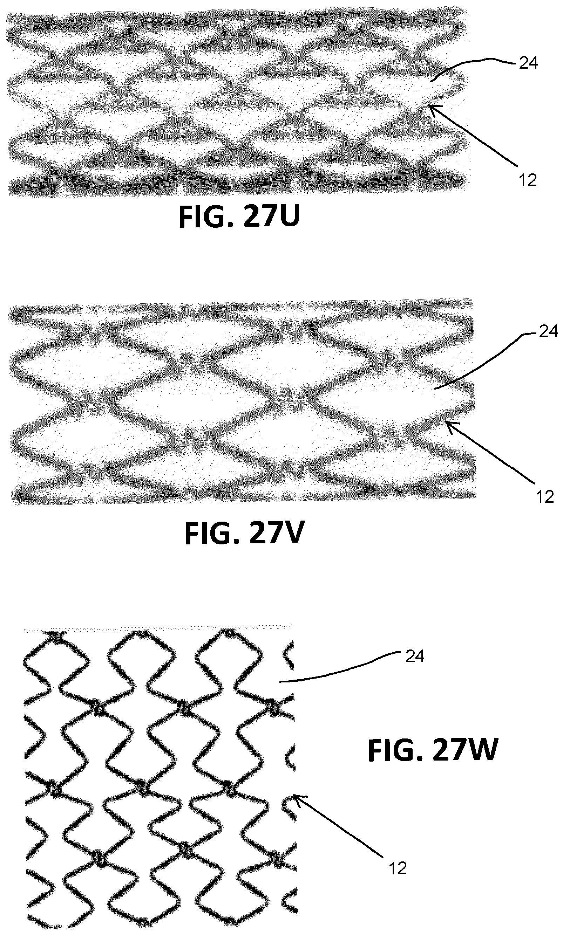

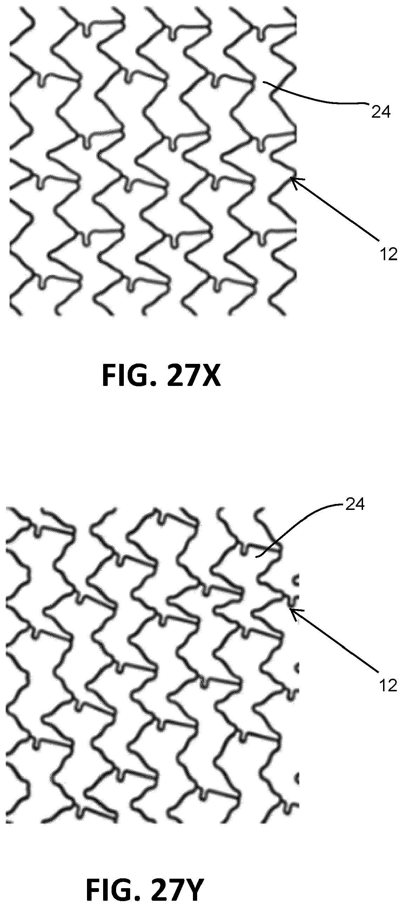

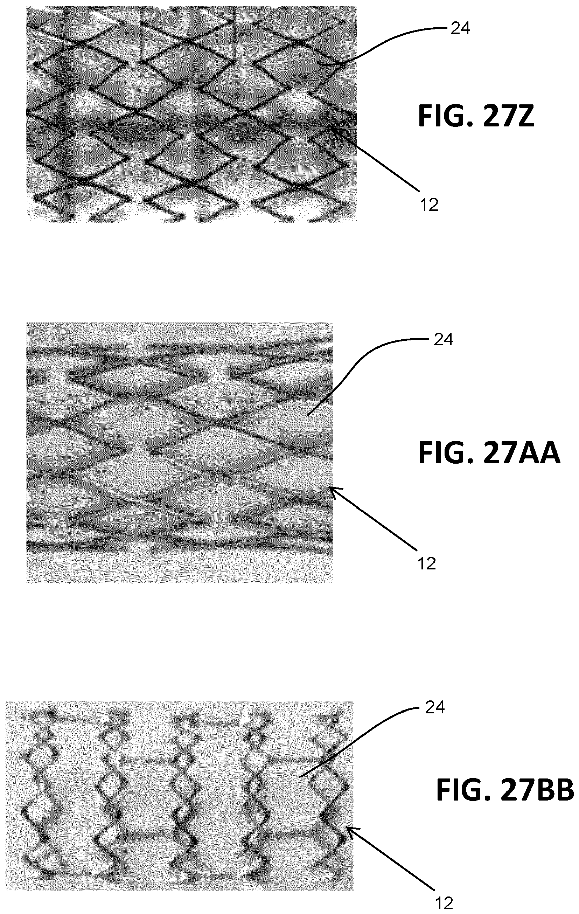

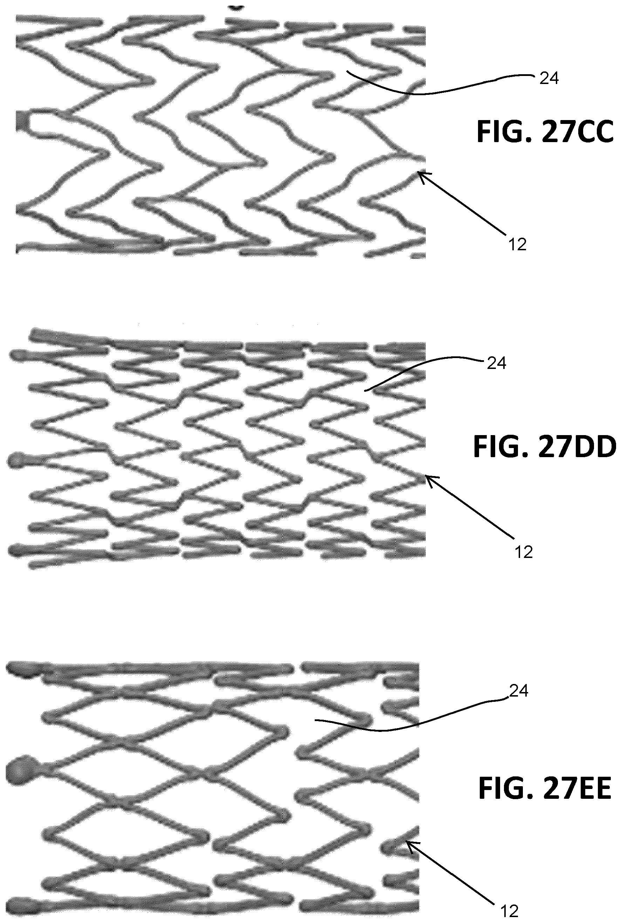

FIGS. 27A and 27B illustrate additional embodiments of helical stents that may be used as reinforcing elements 12 to produce a graft 10 with corrugations 26 as disclosed herein. The reinforcing elements 12 in FIGS. 27A and 27B have similar configurations, having helical crowns 16 with alternating teeth 34, where the teeth 34 of adjacent crowns 16 extend in the same directions relative to each other. However, the reinforcing element 12 in FIG. 27A has sparsely spaced crowns 16, and the reinforcing element 12 in FIG. 27B has closely spaced crowns 16, as described herein. Thus, the reinforcing element 12 in FIG. 27A may be more suitable for use with the method 200 in FIGS. 5 and 7-12, and the reinforcing element 12 in FIG. 27B may be more suitable for use with the method 300 in FIGS. 6 and 13-18.

The graft 10 and methods 100, 200, 300 disclosed herein may also be used in connection with reinforcing elements 12 in the form of laser cut stents, e.g., formed of Nitinol. Such laser cut stents may be formed in a nearly limitless variety of configurations. FIGS. 27C-27EE show various embodiments of laser cut stents that may be used as reinforcing elements 12 in accordance with embodiments disclosed herein.

Various embodiments of grafts and methods for producing the same have been described herein, which include various components and features. In other embodiments, the grafts and methods for producing the same may be provided with any combination of such components and features. It is also understood that in other embodiments, the various devices, components, and features of the grafts or the reinforcing elements thereof described herein may be constructed with similar structural and functional elements having different configurations, including different ornamental appearances.

Several alternative embodiments and examples have been described and illustrated herein. A person of ordinary skill in the art would appreciate the features of the individual embodiments, and the possible combinations and variations of the components. A person of ordinary skill in the art would further appreciate that any of the embodiments could be provided in any combination with the other embodiments disclosed herein. It is understood that the invention may be embodied in other specific forms without departing from the spirit or central characteristics thereof. The present examples and embodiments, therefore, are to be considered in all respects as illustrative and not restrictive, and the invention is not to be limited to the details given herein. Nothing in this specification should be construed as requiring a specific three-dimensional orientation of structures in order to fall within the scope of this invention, unless explicitly specified by the claims. When used in description of a method or process, the term "providing" (or variations thereof) as used herein means generally making an article available for further actions, and does not imply that the entity "providing" the article manufactured, assembled, or otherwise produced the article. The term "approximately" as used herein implies a variation of up to 10% of the nominal value modified by such term, or up to 10% of a midpoint value of a range modified by such term. The term "plurality," as used herein, indicates any number greater than one, either disjunctively or conjunctively, as necessary, up to an infinite number. Accordingly, while the specific embodiments have been illustrated and described, numerous modifications come to mind without significantly departing from the spirit of the invention and the scope of protection is only limited by the scope of the accompanying claims.

The following non-limiting and non-exhaustive description of exemplary embodiments is intended to further describe certain embodiments of the invention.
1. A graft comprising:
   a reinforcing element defining a continuous tubular structure extending axially between first and second ends, the reinforcing element having a natural length between the first and second ends, the reinforcing element having a plurality of gaps therethrough located between the first and second ends; and
   a first film layer fixed to the reinforcing element and extending around the tubular structure, the first film layer extending across at least some of the plurality of gaps;
   wherein the first film layer has a plurality of corrugations within the plurality of gaps when the graft is in an equilibrium state.
2. The graft according to the preceding embodiment, wherein the reinforcing element is a stent.
3. The graft according to any one of the preceding embodiments, wherein the reinforcing element is compressed to a length shorter than the natural length of the reinforcing element when the graft is in the equilibrium state.
4. The graft according to any one of the preceding embodiments, wherein the reinforcing element comprises a helical wire forming a plurality of crowns, wherein the reinforcing element is configured such that a first crown of the plurality of crowns does not circumferentially overlap any adjacent portions of a second crown of the plurality of crowns when the reinforcing element is at the natural length.
5. The graft according to any one of the preceding embodiments, wherein the reinforcing element is extended to a length longer than the natural length of the reinforcing element when the graft is in the equilibrium state.
6. The graft according to any one of the preceding embodiments, wherein the reinforcing element comprises a helical wire forming a plurality of crowns, wherein the reinforcing element is configured such that at least one of the crowns of the plurality of crowns circumferentially overlaps an adjacent portion of a second crown of the plurality of crowns when the reinforcing element is at the natural length.
7. The graft according to any one of the preceding embodiments, further comprising a second film layer fixed to the reinforcing element and extending around the tubular structure, the second film layer extending across at least some of the plurality of gaps, wherein the first film layer is an inner film layer disposed radially inward of the reinforcing element and the second layer is an outer film layer disposed radially outward of the reinforcing element.
8. The graft according to any one of the preceding embodiments, wherein both the first film layer and the second film layer have a plurality of corrugations within the plurality of gaps when the graft is in the equilibrium state.
9. The graft according to any one of the preceding embodiments, wherein at least some of the plurality of gaps are open gaps that extend around at least a full circumference of the graft.
10. The graft according to any one of the preceding embodiments, wherein at least some of the plurality of gaps are closed gaps that extend around less than a full circumference of the graft.
11. The graft according to any one of the preceding embodiments, wherein the reinforcing element comprises a helical winding.
12. The graft according to any one of the preceding embodiments, wherein the reinforcing element comprises a plurality of separate rings connected by bridges.
13. The graft according to any one of the preceding embodiments, wherein the reinforcing element comprises a helical winding with bridges extending between portions of the helical winding.
14. The graft according to any one of the preceding embodiments, wherein the bridges are axially extending.
15. The graft according to any one of the preceding embodiments, wherein the first and/or second film layer is formed of ultrahigh molecular weight polyethylene (UHMWPE).
16. The graft according to any one of the preceding embodiments, wherein the first and/or second film layer has a tensile modulus of at least 100 MPa.
17. The graft according to any one of the preceding embodiments, wherein the reinforcing element is formed of metal wire.
18. The graft according to any one of the preceding embodiments, wherein the reinforcing element is a laser cut stent.
19. The graft according to any one of the preceding embodiments, wherein the reinforcing element is formed of a shape-memory alloy.
20. The graft according to any one of the preceding embodiments, wherein the reinforcing element is formed of Nitinol.
21. The graft according to any one of the preceding embodiments, wherein the reinforcing element is formed of fabric.
22. The graft according to any one of the preceding embodiments, wherein the reinforcing element is formed of silicone.
23. The graft according to any one of the preceding embodiments, wherein the first film layer forms an innermost surface of the graft.
24. The graft according to any one of the preceding embodiments, wherein the second film layer forms an outermost surface of the graft.
25. The graft according to any one of the preceding embodiments, wherein the plurality of corrugations includes at least some regular corrugations.
26. The graft according to any one of the preceding embodiments, wherein the plurality of corrugations includes at least some irregular corrugations.
27. The graft according to any one of the preceding embodiments, further comprising at least a third film layer fixed to the reinforcing element and extending around the tubular structure.
28. The graft according to any one of the preceding embodiments, wherein the second film layer is a helical strip.
29. The graft according to any one of the preceding embodiments, wherein the reinforcing element extends substantially an entire length of the graft.
30. The graft according to any one of the preceding embodiments, wherein the reinforcing element extends less than an entire length of the graft.
31. The graft according to any one of the preceding embodiments, wherein the reinforcing element comprises a mesh material.
32. A method of manufacturing a corrugated graft, comprising:
   positioning a reinforcing element on a mandrel to define a continuous tubular structure extending axially between first and second ends, the reinforcing element having a natural length between the first and second ends, the reinforcing element having a plurality of gaps therethrough located between the first and second ends;
   positioning a first film layer in contact with the reinforcing element on the mandrel and extending around the tubular structure;
   thermally bonding the first film layer to the reinforcing element to fix the first film layer to the reinforcing element and form a graft, such that the first film layer extends across at least some of the plurality of gaps;
   exerting an axial force on the reinforcing element to change a length of the reinforcing element such that the reinforcing element has a modified length that is different from the natural length; and
   releasing the axial force on the reinforcing element, permitting the reinforcing element to relax toward the natural length thereof, creating a corrugated graft where the first film layer has a plurality of corrugations within the plurality of gaps when the corrugated graft is in an equilibrium state.
33. The method according to the preceding embodiment, wherein the reinforcing element is a stent.
34. The method according to any one of the preceding embodiments, wherein the axial force is a tensile force, and the modified length is longer than the natural length, and wherein the plurality of corrugations are formed by the reinforcing element axially compressing the first film layer as the reinforcing element decreases in length from the modified length toward the natural length, wherein optionally an additional axial compressive force is applied to the reinforcing element after releasing the tensile force, to decrease the length of the reinforcing element from the modified length.
35. The method according to any one of the preceding embodiments, wherein the axial force is exerted on the reinforcing element prior to thermally bonding the first film layer to the reinforcing element, such that the reinforcing element is in the modified length during the thermal bonding.
36. The method according to any one of the preceding embodiments, wherein when the corrugated graft is in the equilibrium state, an equilibrium length of the stent remains longer than the natural length.
37. The method according to any one of the preceding embodiments, wherein the reinforcing element comprises a helical wire forming a plurality of crowns, wherein at least one of the crowns of the plurality of crowns circumferentially overlaps an adjacent portion of a second crown of the plurality of crowns when the reinforcing element is at the natural length.
38. The method according to any one of the preceding embodiments, wherein the axial force is a compressive force, and the modified length is shorter than the natural length, and wherein the plurality of corrugations are formed by the reinforcing element axially compressing the first film layer when the axial force compresses the reinforcing element from the natural length to the modified length.
39. The method according to any one of the preceding embodiments, wherein the axial force is exerted on the reinforcing element after thermally bonding the first film layer to the reinforcing element, such that the axial force is exerted on both the reinforcing element and the first film layer.
40. The method according to any one of the preceding embodiments, wherein when the corrugated graft is in the equilibrium state, an equilibrium length of the reinforcing element remains shorter than the natural length.
41. The method according to any one of the preceding embodiments, wherein the reinforcing element comprises a helical wire forming a plurality of crowns, wherein a first crown of the plurality of crowns does not circumferentially overlap any adjacent portions of a second crown of the plurality of crowns when the reinforcing element is at the natural length.
42. The method according to any one of the preceding embodiments, further comprising:
   positioning a second film layer in contact with the reinforcing element on the mandrel and extending around the tubular structure, wherein the first film layer is an inner film layer disposed radially inward of the reinforcing element and the second layer is an outer film layer disposed radially outward of the reinforcing element; and
   thermally bonding the second film layer to the reinforcing element to fix the second film layer to the reinforcing element, such that the second film layer extends across at least some of the plurality of gaps,
   wherein the second film layer has a plurality of corrugations within the plurality of gaps when the corrugated graft is in the equilibrium state.
43. The method according to any one of the preceding embodiments, wherein thermally bonding the first film layer and the second film layer to the reinforcing element bonds the first film layer to the second film layer to fix the first and second film layers to the reinforcing element.
44. The method according to any one of the preceding embodiments, wherein after thermally bonding the first film layer to the stent and prior to exerting the axial force on the graft, the method further comprises placing the graft on a second mandrel having a larger circumference than the mandrel.
45. The method according to any one of the preceding embodiments, wherein at least some of the plurality of gaps are open gaps that extend around at least a full circumference of the graft.
46. The method according to any one of the preceding embodiments, wherein at least some of the plurality of gaps are closed gaps that extend around less than a full circumference of the graft.
47. The method according to any one of the preceding embodiments, wherein the stent comprises a helical winding.
48. The method according to any one of the preceding embodiments, wherein the stent comprises a plurality of separate windings connected by bridges.
49. The method according to any one of the preceding embodiments, wherein the stent comprises a helical winding with bridges extending between portions of the helical winding.
50. The method according to any one of the preceding embodiments, wherein the bridges are axially extending.
51. The method according to any one of the preceding embodiments, wherein the first and/or second film layer is formed of ultrahigh molecular weight polyethylene (UHMWPE).
52. The method according to any one of the preceding embodiments, wherein the first and/or second film layer has a tensile modulus of at least 100 MPa.
53. The method according to any one of the preceding embodiments, wherein the stent is formed of metal wire.
54. The method according to any one of the preceding embodiments, wherein the stent is a laser cut stent.
55. The method according to any one of the preceding embodiments, wherein the stent is formed of a shape-memory alloy.
56. The method according to any one of the preceding embodiments, wherein the stent is formed of Nitinol.
57. The method according to any one of the preceding embodiments, wherein the stent is formed of fabric.
58. The method according to any one of the preceding embodiments, wherein the stent is formed of silicone.
59. The method according to any one of the preceding embodiments, wherein the first film layer forms an innermost surface of the graft.
60. The method according to any one of the preceding embodiments, wherein the second film layer forms an outermost surface of the graft.
61. The method according to any one of the preceding embodiments, wherein the plurality of corrugations includes at least some regular corrugations.
62. The method according to any one of the preceding embodiments, wherein the plurality of corrugations includes at least some irregular corrugations.
63. The method according to any one of the preceding embodiments, further comprising at least a third film layer fixed to the stent and extending around the tubular structure.
64. The method according to any one of the preceding embodiments, wherein the second film layer is a helical strip.
65. The method according to any one of the preceding embodiments, wherein the reinforcing element extends substantially an entire length of the graft.
66. The method according to any one of the preceding embodiments, wherein the reinforcing element extends less than an entire length of the graft.
67. The method according to any one of the preceding embodiments, wherein the reinforcing element comprises a mesh material.
68. A method of manufacturing a corrugated graft, comprising:
   positioning a reinforcing element on a mandrel to form a continuous tubular structure extending axially between first and second ends, the reinforcing element having a natural length between the first and second ends, the reinforcing element having a plurality of gaps therethrough located between the first and second ends;
   positioning a first film layer in contact with the reinforcing element on the mandrel and extending around the tubular structure;
   thermally bonding the first film layer to the reinforcing element to fix the first film layer to the reinforcing element and form a graft, such that the first film layer extends across at least some of the plurality of gaps; and
   reducing a length of the reinforcing element after the thermal bonding, thereby axially compressing the first film layer and forming a plurality of corrugations in the first film layer within the plurality of gaps when the graft is in an equilibrium state, creating a corrugated graft.
69. The method according to the preceding embodiment, wherein the reinforcing element is a stent.
70. The method according to any one of the preceding embodiments, further comprising exerting a tensile axial force on the reinforcing element to increase the length of the reinforcing element to a modified length that is longer than the natural length prior to the thermal bonding, and wherein the plurality of corrugations are formed by axially compressing the first film layer as the length of the reinforcing element is reduced from the modified length toward the natural length.
71. The method according to any one of the preceding embodiments, wherein the length of the reinforcing element is reduced by exerting a compressive axial force on the reinforcing element subsequent to the thermal bonding.
72. The method according to any one of the preceding embodiments, wherein when the corrugated graft is in the equilibrium state, an equilibrium length of the stent remains longer than the natural length.
73. The method according to any one of the preceding embodiments, wherein the reinforcing element comprises a helical wire forming a plurality of crowns, wherein at least one of the crowns of the plurality of crowns circumferentially overlaps an adjacent portion of a second crown of the plurality of crowns when the reinforcing element is at the natural length.
74. The method according to any one of the preceding embodiments, wherein the length of the reinforcing element is reduced by exerting a compressive axial force on the reinforcing element after thermally bonding the first film layer to the reinforcing element, and wherein the plurality of corrugations are formed by axially compressing the first film layer when the compressive axial force compresses the reinforcing element.
75. The method according to any one of the preceding embodiments, wherein the thermal bonding is conducted with the reinforcing element at the natural length.
76. The method according to any one of the preceding embodiments, wherein when the corrugated graft is in the equilibrium state, an equilibrium length of the reinforcing element is shorter than the natural length.
77. The method according to any one of the preceding embodiments, wherein the reinforcing element comprises a helical wire forming a plurality of crowns, wherein a first crown of the plurality of crowns does not circumferentially overlap any adjacent portions of a second crown of the plurality of crowns when the reinforcing element is at the natural length.
78. The method of according to any one of the preceding embodiments, further comprising:
   positioning a second film layer in contact with the reinforcing element on the mandrel and extending around the tubular structure, wherein the first film layer is an inner film layer disposed radially inward of the reinforcing element and the second layer is an outer film layer disposed radially outward of the reinforcing element; and
   thermally bonding the second film layer to the reinforcing element to fix the second film layer to the reinforcing element, such that the second film layer extends across at least some of the plurality of gaps,
   wherein reducing the length of the reinforcing element further axially compresses the second film layer and forms a plurality of corrugations in the second film layer within the plurality of gaps when the corrugated graft is in the equilibrium state.
79. The method according to any one of the preceding embodiments, wherein thermally bonding the first film layer and the second film layer to the reinforcing element bonds the first film layer to the second film layer to fix the first and second film layers to the reinforcing element.
80. The method according to any one of the preceding embodiments, wherein at least some of the plurality of gaps are open gaps that extend around at least a full circumference of the graft.
81. The method according to any one of the preceding embodiments, wherein at least some of the plurality of gaps are closed gaps that extend around less than a full circumference of the graft.
82. The method according to any one of the preceding embodiments, wherein the stent comprises a helical winding.
83. The method according to any one of the preceding embodiments, wherein the stent comprises a plurality of separate windings connected by bridges.
84. The method according to any one of the preceding embodiments, wherein the stent comprises a helical winding with bridges extending between portions of the helical winding.
85. The method according to any one of the preceding embodiments, wherein the bridges are axially extending.
86. The method according to any one of the preceding embodiments, wherein the first and/or second film layer is formed of ultrahigh molecular weight polyethylene (UHMWPE).
87. The method according to any one of the preceding embodiments, wherein the first and/or second film layer has a tensile modulus of at least 100 MPa.
88. The method according to any one of the preceding embodiments, wherein the stent is formed of metal wire.
89. The method according to any one of the preceding embodiments, wherein the stent is a laser cut stent.
90. The method according to any one of the preceding embodiments, wherein the stent is formed of a shape-memory alloy.
91. The method according to any one of the preceding embodiments, wherein the stent is formed of Nitinol.
92. The method according to any one of the preceding embodiments, wherein the stent is formed of fabric.
93. The method according to any one of the preceding embodiments, wherein the stent is formed of silicone.
94. The method according to any one of the preceding embodiments, wherein the first film layer forms an innermost surface of the graft.
95. The method according to any one of the preceding embodiments, wherein the second film layer forms an outermost surface of the graft.
96. The method according to any one of the preceding embodiments, wherein the plurality of corrugations includes at least some regular corrugations.
97. The method according to any one of the preceding embodiments, wherein the plurality of corrugations includes at least some irregular corrugations.
98. The method according to any one of the preceding embodiments, further comprising at least a third film layer fixed to the stent and extending around the tubular structure.
99. The method according to any one of the preceding embodiments, wherein the second film layer is a helical strip.
100. The method according to any one of the preceding embodiments, wherein the reinforcing element extends substantially an entire length of the graft.
101. The method according to any one of the preceding embodiments, wherein the reinforcing element extends less than an entire length of the graft.
102. The method according to any one of the preceding embodiments, wherein the reinforcing element comprises a mesh material.

## Claims

1. A graft comprising:
a reinforcing element defining a continuous tubular structure extending axially between first and second ends, the reinforcing element having a natural length between the first and second ends, the reinforcing element having a plurality of gaps therethrough located between the first and second ends; and
a first film layer fixed to the reinforcing element and extending around the tubular structure, the first film layer extending across at least some of the plurality of gaps,
wherein the first film layer has a plurality of corrugations within the plurality of gaps when the graft is in an equilibrium state.

2. The graft of claim 1, wherein the reinforcing element is a stent.

3. The graft of claim 1 or 2, wherein the reinforcing element is compressed to a length shorter than the natural length of the reinforcing element when the graft is in the equilibrium state.

4. The graft of claim 3, wherein the reinforcing element comprises a helical wire forming a plurality of crowns, wherein the reinforcing element is configured such that a first crown of the plurality of crowns does not circumferentially overlap any adjacent portions of a second crown of the plurality of crowns when the reinforcing element is at the natural length.

5. The graft of any one of claims 1-4, wherein the reinforcing element is extended to a length longer than the natural length of the reinforcing element when the graft is in the equilibrium state.

6. The graft of claim 5, wherein the reinforcing element comprises a helical wire forming a plurality of crowns, wherein the reinforcing element is configured such that at least one of the crowns of the plurality of crowns circumferentially overlaps an adjacent portion of a second crown of the plurality of crowns when the reinforcing element is at the natural length.

7. The graft of any one of claims 1-6, further comprising a second film layer fixed to the reinforcing element and extending around the tubular structure, the second film layer extending across at least some of the plurality of gaps, wherein the first film layer is an inner film layer disposed radially inward of the reinforcing element and the second layer is an outer film layer disposed radially outward of the reinforcing element.

8. The graft of claim 7, wherein both the first film layer and the second film layer have a plurality of corrugations within the plurality of gaps when the graft is in the equilibrium state.

9. The graft of any one of claims 1-8, wherein at least some of the plurality of gaps are open gaps that extend around at least a full circumference of the graft.

10. The graft of any one of claims 1-9, wherein at least some of the plurality of gaps are closed gaps that extend around less than a full circumference of the graft.

11. A method of manufacturing a corrugated graft, comprising:
positioning a reinforcing element on a mandrel to form a continuous tubular structure extending axially between first and second ends, the reinforcing element having a natural length between the first and second ends, the reinforcing element having a plurality of gaps therethrough located between the first and second ends;
positioning a first film layer in contact with the reinforcing element on the mandrel and extending around the tubular structure;
thermally bonding the first film layer to the reinforcing element to fix the first film layer to the reinforcing element and form a graft, such that the first film layer extends across at least some of the plurality of gaps; and
reducing a length of the reinforcing element after the thermal bonding, thereby axially compressing the first film layer and forming a plurality of corrugations in the first film layer within the plurality of gaps when the graft is in an equilibrium state, creating a corrugated graft.

12. The method of claim 11, further comprising exerting a tensile axial force on the reinforcing element to increase the length of the reinforcing element to a modified length that is longer than the natural length prior to the thermal bonding, and wherein the plurality of corrugations are formed by axially compressing the first film layer as the length of the reinforcing element is reduced from the modified length toward the natural length.

13. The method of claim 12, wherein the length of the reinforcing element is reduced by exerting a compressive axial force on the reinforcing element subsequent to the thermal bonding.

14. The method of claim 12 or 13, wherein the reinforcing element comprises a helical wire forming a plurality of crowns, wherein at least one of the crowns of the plurality of crowns circumferentially overlaps an adjacent portion of a second crown of the plurality of crowns when the reinforcing element is at the natural length.

15. The method of any one of claims 11-14, further comprising:
positioning a second film layer in contact with the reinforcing element on the mandrel and extending around the tubular structure, wherein the first film layer is an inner film layer disposed radially inward of the reinforcing element and the second layer is an outer film layer disposed radially outward of the reinforcing element; and
thermally bonding the second film layer to the reinforcing element to fix the second film layer to the reinforcing element, such that the second film layer extends across at least some of the plurality of gaps,
wherein reducing the length of the reinforcing element further axially compresses the second film layer and forms a plurality of corrugations in the second film layer within the plurality of gaps when the corrugated graft is in the equilibrium state.
